# EUROPÄISCHE PATENTANMELDUNG

(11) **EP 1 149 604 A1**
(43) Veröffentlichungstag der Anmeldung: **31.10.2001**
(21) Anmeldenummer: 01105580.3
(22) Anmeldetag: 06.03.2001
(51) Int. Cl.: A61M 25/00

(54) **Harnleiterkatheter zur Applikation einer Flüssigkeit in einen Harnleiter**

(30) Priorität: 28.04.2000 DE 20007733 U
(71) Anmelder: Thiel, Ulrich, Dr. med., 16341 Cepernick (DE)
(72) Erfinder: Thiel, Ulrich, Dr. med., 16341 Cepernick (DE)
(74) Vertreter: Hofstetter, Alfons J., Dr.rer.nat.

(57) **Zusammenfassung**

Die vorliegende Erfindung betrifft einen Harnleiterkatheter (10) zur Applikation einer Flüssigkeit in einen Harnleiter, mit einem Katheterschaft (12), wobei der Katheterschaft (12) ein proximales Ende (16) und ein distales Ende (18) mit einer Katheterspitze (20) aufweist, wobei die Flüssigkeit von dem proximalen Ende (16) zu dem distalen Ende (18) des Katheterschafts (12) transportierbar ist, und der Harnleiterkatheter (10) in einem Bereich des distalen Endes (18) des Katheterschafts (12) mindestens eine Öffnung (22) aufweist, durch die eine Flüssigkeit aus dem Katheterschaft (12) jeweils in mindestens eine Richtung ausführbar ist, die aus einer ersten Richtungskomponente parallel zur Einführrichtung des Katheterschafts (12) und einer zweiten Richtungskomponente senkrecht zur ersten Richtungskomponente zusammengesetzt ist.

## Beschreibung

Die Erfindung betrifft einen Harnleiterkatheter zur Applikation einer Flüssigkeit in einen Harnleiter, mit einem Katheterschaft, wobei der Katheterschaft ein proximales und ein distales Ende mit einer Katheterspitze aufweist, wobei die Flüssigkeit von dem proximalen Ende zu dem distalen Ende des Katheterschafts transportierbar ist.

Ein derartiger Harnleiterkatheter ist bereits bekannt und weist im Zentrum der Katheterspitze eine Öffnung auf, durch die die Flüssigkeit aus dem Katheterschaft austreten kann. Ein solcher Harnleiterkatheter kann, nachdem er in den Harnleiter eingeführt wurde, dazu verwendet werden, beispielsweise ein Arzneimittel in den Harnleiter zu applizieren. Ein derartiger Harnleiterkatheter findet jedoch auch bei der Behandlung von Harnleitersteinen Anwendung. Durch die aus dem Harnleiterkatheter durch die zentrale Öffnung ausströmende Flüssigkeit kann ein im Harnleiter befindliches Konkrement gelöst werden, so daß dieses entweder durch eine Extraktion entfernt werden kann oder durch eine Reposition in das Nierenbecken hochgespült werden kann.

Nachteilig an diesem Harnleiterkatheter ist jedoch, daß der Harnleiterkatheter insbesondere bei bogenförmigen oder in Schlingen verlaufenden Harnleitern oder auch bei vorliegenden Harnleiterverengungen bzw. Harnleiterstenosen extrem schwer einzuführen ist und daher häufig Harnleiterverletzungen verursacht. Auch bei dem Lösen eines im Harnleiter festsitzenden Konkrements durch die aus dem Harnleiterkatheter ausströmende Flüssigkeit kann es zu Harnleiterperforationen oder sogar zu einem Harnleiterabriß kommen.

Aufgabe der vorliegenden Erfindung ist es daher, einen Harnleiterkatheter der eingangs genannten Art bereitzustellen, der eine leichte Einführbarkeit in den Harnleiter, eine leichte Verschiebbarkeit innerhalb des Harnleiters und ein sicheres Lösen von Hamleiterkonkrementen ermöglicht.

Zur Lösung dieser Aufgabe dienen die Merkmale des unabhängigen Anspruchs 1.

Vorteilhafte Ausgestaltungen sind in den Unteransprüchen beschrieben.

Der erfindungsgemäße Harnleiterkatheter weist in einem Bereich des distalen Endes des Katheterschafts mindestens eine Öffnung auf. Diese mindestens eine Öffnung ist jedoch so ausgeführt, daß durch sie ausströmende Flüssigkeit in eine Richtung gelenkt wird, die eine erste Richtungskomponente parallel zur Einführrichtung des Katheterschafts und eine zweite Richtungskomponente senkrecht zur ersten Richtungskomponente besitzt. Auf diese Weise sorgt der Flüssigkeitsstrom nicht nur dafür, daß ein Druck in Einführrichtung des Harnleiterkatheters ausgeübt wird, sondern auch dafür, daß der Harnleiter in unmittelbarer Umgebung des distalen Endes des Katheterschafts durch die herausströmende Flüssigkeit vorsichtig gedehnt, bzw. geweitet wird. Der erfindungsgemäße Harnleiterkatheter läßt sich auf diese Weise extrem leicht innerhalb des Harnleiters verschieben und minimiert dadurch die Verletzungsgefahr außerordentlich. Durch diese sanfte Weitung des Harnleiters in Einführrichtung des Harnleiterkatheters lassen sich auch im Harnleiter verkeilte Konkremente sehr viel leichter lösen.

In einer ersten vorteilhaften Weiterbildung der Erfindung beträgt der Durchmesser des Katheterschafts 5 F bis 7 F, insbesondere 6 F. Aufgrund dieses geringen Durchmessers kann der Harnleiterkatheter auch in verengte Harnleiter eingeführt werden.

Der Katheterschaft kann zwischen 50 cm und 70 cm, insbesondere 60 cm lang sein.

In einer weiteren vorteilhaften Weiterbildung der Erfindung ist die Katheterspitze ellipsoidförmig ausgebildet. Durch eine derartige Ellipsoidform wird die Dehnung des Harnleiters in der Umgebung des distalen Endes des Katheterschafts zusätzlich verstärkt. Die Katheterspitze kann 0,5 cm bis 2 cm, insbesondere jedoch 1 cm lang sein.

In einer weiteren vorteilhaften Weiterbildung der Erfindung beträgt der maximale Durchmesser der Katheterspitze 8 F.

Zudem kann die mindestens eine Öffnung in der Katherspitze angeordnet sein. Auf diese Weise kann der Harnleiter im Bereich der Katheterspitze und unmittelbar vor der Katheterspitze erweitert werden.

In einer weiteren vorteilhaften Ausgestaltung der Erfindung ist die mindestens eine Öffnung in einer Ebene orthogonal zur Einführrichtung des Katheterschafts angeordnet. Dabei kann die mindestens eine Öffnung mindestens drei Öffnungen, insbesondere vier bis sechs Öffnungen umfassen, wobei die Öffnungen äquidistant angeordnet sind. Eine solche gleichmäßige Verteilung mehrerer Öffnungen gewährleistet eine ebenso gleichmäßige Erweiterung des Harnleiters.

Die Flüssigkeit kann auch von dem proximalen Ende zu dem distalen Ende des Katheterschafts durch ein Flüssigkeitleitelement leitbar sein. Diese Weiterbildung ist insbesondere dann von Vorteil, wenn gleichzeitig ein Führungsdraht und/oder optische Elemente durch den Katheterschaft hindurchgeführt werden müssen und eine Benetzung dieser Elemente durch die zu leitende Flüssigkeit unerwünscht ist.

Der erfindungsgemäße Harnleiterkatheter kann auch eine weitere Öffnung an dem distalen Ende des Katheterschafts, insbesondere in der Katheterspitze, aufweisen, wobei durch die weitere Öffnung ein Führungsdraht führbar ist. Diese weitere Öffnung kann zentral in der Katheterspitze angeordnet sein.

Weitere Einzelheiten, Merkmale und Vorteile des erfindungsgemäßen Harnleiterkatheters ergeben sich aus der folgenden Beschreibung zeichnerisch dargestellter Ausführungsbeispiele.

Es zeigen:
- Fig. 1: eine schematische Darstellung einer ersten Ausführungsform eines erfindungsgemäßen Harnleiterkatheters;
- Fig. 2: einen schematisch dargestellten Längsschnitt durch das distale Ende der ersten Ausführungsform des erfindungsgemäßen Harnleiterkatheters;
- Fig. 3: eine schematische Aufsicht auf eine Katheterspitze der ersten Ausführungsform des erfindungsgemäßen Katheters;
- Fig. 4: eine schematische Darstellung eines distalen Endes einer zweiten Ausführungsform des erfindungsgemäßen Harnleiterkatheters;
- Fig. 5: eine schematische Aufsicht auf eine Katheterspitze der zweiten Ausführungsform des erfindungsgemäßen Harnleiterkatheters.

Gleiche Bauteile weisen in den Figuren die gleichen Bezugszahlen auf.

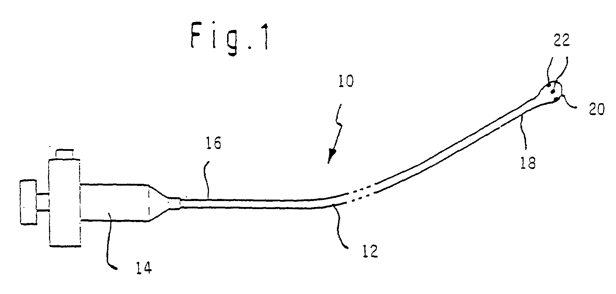

Fig. 1 zeigt eine schematische Darstellung einer ersten Ausführungsform des erfindungsgemäßen Harnleiterkatheters 10. Der Harnleiterkatheter 10 weist einen Katheterschaft 12 und ein Betätigungselement 14 auf, wobei das proximale Ende 16 des Katheterschafts 12 mit dem Betätigungselement 14 verbunden ist. Ein distales Ende 18 des Katheterschafts 12 weist eine ellipsoidförmige Katheterspitze 20 mit sechs Öffnungen 22 auf. Der Durchmesser des Katheterschafts 12 beträgt 6 F, während der maximale Durchmesser der Katheterspitze 20 8 F beträgt.

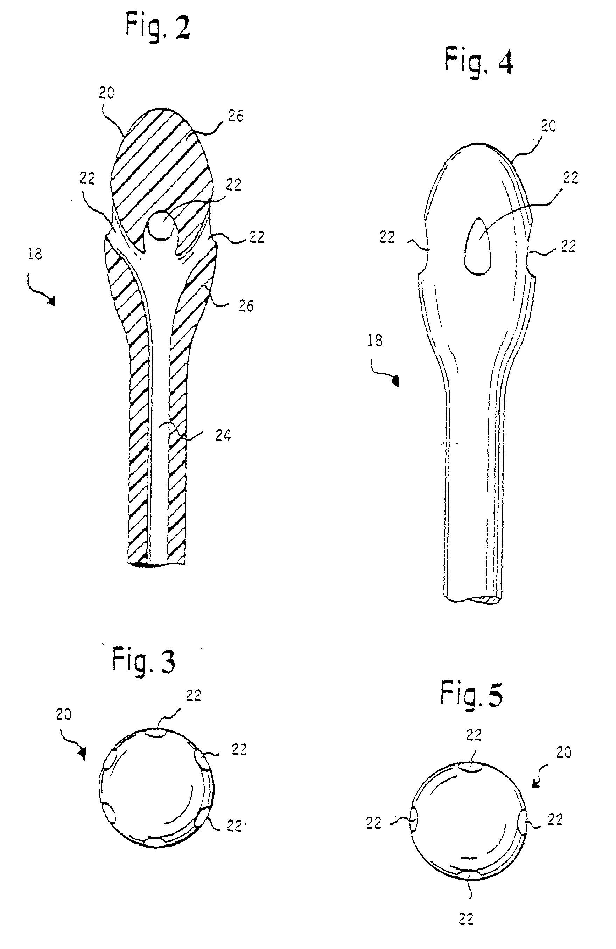

Fig. 2 zeigt das distale Ende 18 des Katheterschafts 12 in einer schematisch dargestellten Schnittzeichnung entlang der Längsachse des Katheterschafts 12. Eine Flüssigkeit kann nun über den Hohlraum 24 des Katheterschafts 12 transportiert werden und durch die Öffnungen 22, die schräg durch die Wand 26 der Katheterspitze 20 treten, aus dem Harnleiterkatheter 10 herausgeführt werden. Diese Ausführungsform der Erfindung ist besonders geeignet zur Entfernung von Harnleitersteinen, da durch den schrägen, cranialwärts gerichteten Flüssigkeitsstrom das Loslösen eines Harnleitersteines enorm vereinfacht wird.

In Fig. 3 ist eine schematische Aufsicht auf die Katheterspitze 20 mit den sechs Öffnungen 22 dargestellt.

Fig. 4 zeigt das distale Ende 18 einer zweiten Ausführungsform des Harnleiterkatheters 10, der nur vier Öffnungen 22 aufweist, die jedoch auch äquidistant zueinander in einer Ebene orthogonal zu einer Einführrichtung des Katheterschafts 12 angeordnet sind.

In Fig. 5 ist eine schematische Aufsicht auf die Katheterspitze 20 dieser zweiten Ausführungsform des Harnleiterkatheters 10 dargestellt.

Eine weitere, nicht dargestellte Ausführungsform des erfindungsgemäßen Harnleiterkatheters weist nur drei Öffnungen auf, die allerdings jeweils einen Öffnungswinkel von bis zu 110° besitzen, so daß auch hier eine äußerst gleichmäßige Dehnung des Harnleiters erreicht werden kann.

Eine weitere Ausführungsform weist eine zusätzliche zentrale Öffnung auf, durch die ein Führungsdraht aus dem Katheterschaft herausgeführt werden kann. Flüssigkeiten werden bei eingelegtem Führungsdraht aber auch hier, zumindest vorwiegend, durch die seitlichen Öffnungen aus dem Katheterschaft herausgeführt.

## Patentansprüche

1. Harnleiterkatheter (10) zur Applikation einer Flüssigkeit in einen Harnleiter, mit einem Katheterschaft (12),
wobei der Katheterschaft (12) ein proximales Ende (16) und ein distales Ende (18) mit einer Katheterspitze (20) aufweist,
wobei die Flüssigkeit von dem proximalen Ende (16) zu dem distalen Ende (18) des Katheterschafts (12) transportierbar ist,
**dadurch gekennzeichnet,**
**daß** der Harnleiterkatheter (10) in einem Bereich des distalen Endes (18) des Katheterschafts (12) mindestens eine Öffnung (22) aufweist, durch die eine Flüssigkeit aus dem Katheterschaft (12) jeweils in mindestens eine Richtung ausführbar ist, die aus einer ersten Richtungskomponente parallel zur Einführrichtung des Katheterschafts (12) und einer zweiten Richtungskomponente senkrecht zur ersten Richtungskomponente zusammengesetzt ist.

2. Harnleiterkatheter nach Anspruch 1,
**dadurch gekennzeichnet,**
**daß** der Durchmesser des Katheterschafts (12) 5F bis 7F, insbesondere 6F, beträgt

3. Harnleiterkatheter nach einem der vorhergehenden Ansprüche,
**dadurch gekennzeichnet,**
**daß** der Katheterschaft (12) zwischen 50 cm und 70 cm, insbesondere 60 cm lang ist.

4. Harnleiterkatheter nach einem der vorhergehenden Ansprüche **dadurch gekennzeichnet,**
**daß** die Katheterspitze (20) ellipsoidförmig ausgebildet ist.

5. Hamleiterkatheter nach einem der vorhergehenden Ansprüche,
**dadurch gekennzeichnet,**
**daß** die Katheterspitze (20) 0,5 cm bis 2 cm, insbesondere 1 cm, lang ist.

6. Harnleiterkatheter nach einem der vorhergehenden Ansprüche,
**dadurch gekennzeichnet,**
**daß** der maximale Durchmesser der Katheterspitze (20) 8F beträgt.

7. Harnleiterkatheter nach einem der vorhergehenden Ansprüche,
**dadurch gekennzeichnet,**
**daß** die mindestens eine Öffnung (22) in der Katheterspitze (20) angeordnet ist.

8. Harnleiterkatheter nach einem der vorhergehenden Ansprüche,
**dadurch gekennzeichnet,**
**daß** die mindestens eine Öffnung (22) in einer Ebene orthogonal zur Einführrichtung des Katheterschafts (12) angeordnet ist.

9. Harnleiterkatheter nach Anspruch 8,
**dadurch gekennzeichnet,**
**daß** die mindestens eine Öffnung (22) mindestens drei Öffnungen, insbesondere 4 bis 6 Öffnungen, umfaßt, die äquidistant angeordnet sind.

10. Harnleiterkatheter nach einem der vorhergehenden Ansprüche,
**dadurch gekennzeichnet,**
**daß** die Flüssigkeit von dem proximalen Ende (16) zu dem distalen Ende (18) des Katheterschafts (12) durch ein Flüssigkeitleitelement leitbar ist.

11. Harnleiterkatheter nach einem der vorhergehenden Ansprüche,
**dadurch gekennzeichnet,**
**daß** der Harnleiterkatheter (10) eine weitere Öffnung an dem distalen Ende (18) des Katheterschafts (12), insbesondere in der Katheterspitze (20), aufweist, wobei durch die weitere Öffnung ein Führungsdraht führbar ist.

12. Harnleiterkatheter nach Anspruch 11,
**dadurch gekennzeichnet,**
**daß** die weitere Öffnung zentral in der Katheterspitze (20) angeordnet ist.
